(19) Europäisches Patentamt
European Patent Office
Office européen des brevets

(11) **EP 4 257 598 A1**

(12) **EUROPEAN PATENT APPLICATION**
published in accordance with Art. 153(4) EPC

(43) Date of publication:
**11.10.2023 Bulletin 2023/41**

(51) International Patent Classification (IPC):
*C07K 14/605* (2006.01)      *C07K 16/28* (2006.01)
*A61K 38/00* (2006.01)       *A61P 19/02* (2006.01)
*A23L 33/18* (2016.01)

(21) Application number: 21900764.8

(22) Date of filing: **29.07.2021**

(52) Cooperative Patent Classification (CPC):
**A23L 33/18; A61K 38/00; A61P 19/02;
C07K 14/605; C07K 16/28**

(86) International application number:
**PCT/KR2021/009934**

(87) International publication number:
**WO 2022/119076 (09.06.2022 Gazette 2022/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.12.2020 KR 20200167844**

(71) Applicant: **Immunoforge Co., Ltd.
Seoul 08826 (KR)**

(72) Inventors:
• **CHOI, Hyojung
  Incheon 21997 (KR)**
• **KWON, Hyunjin
  Incheon 21564 (KR)**
• **AHN, Sungmin
  Seoul 06581 (KR)**
• **CHANG, Kiho
  Incheon 22002 (KR)**

(74) Representative: **Icosa
83 avenue Denfert-Rochereau
75014 Paris (FR)**

Remarks:
The complete document including Reference
Table(s) and the Sequence Listing(s) can be
downloaded from the EPO website

(54) **FUSION PROTEIN INCLUDING GLP-1 RECEPTOR AGONIST AND ANTI-OSCAR ANTIBODY, AND USE THEREOF**

(57)    Provided are a fusion protein including a glucagon-like peptide-1 (GLP-1) receptor agonist and an anti-osteoclast-associated receptor (OSCAR) antibody; a pharmaceutical composition for preventing or treating arthritis including the fusion protein; a food composition; a health functional food composition; and a method of preventing or treating arthritis, the method including administering the fusion protein. The fusion protein including a GLP-1 receptor agonist and an anti-OSCAR antibody of the present invention has excellent effects on protecting cartilage and relieving pain and thus may be widely used for treatment of arthritis.

[FIG. 1]

**Description**

**[Technical Field]**

The present invention relates to a fusion protein including a glucagon-like peptide-1 (GLP-1) receptor agonist and an anti-osteoclast-associated receptor (OSCAR) antibody; a pharmaceutical composition for preventing or treating arthritis including the fusion protein; a food composition; a health functional food composition; and a method of preventing or treating arthritis, the method including administering the pharmaceutical composition.

**[Background Art]**

Osteoarthritis is a disease accompanied by local degenerative changes resulting from breakdown of joint cartilage and deformation of joints without inflammatory changes as people become older, and is also called degenerative arthritis due to these characteristics. Although it was commonly thought that osteoarthritis is a natural phenomenon caused as cartilage continuously wears away due to deterioration of all functions of the human body with age in the past, it has recently been revealed that cartilage is deformed and destroyed by various biological factors along with such mechanical factors. Current treatment for osteoarthritis aims to relieve joint pain and restore functions of joints by suppressing causative factors as much as possible so that cartilage denaturation does not progress any further since cartilage loss and joint deformation are caused without inflammatory changes, unlike rheumatoid arthritis.

In the treatment of osteoarthritis, non-steroidal anti-inflammatory drugs (NSAIDs) are commonly prescribed in the market for pain relief and inflammation control in patients. Examples of drugs used to treat osteoarthritis may include simple analgesics, non-steroidal anti-inflammatory drugs, COX-2 selective inhibitors, narcotic and nonnarcotic analgesics, intra-articular steroid injection, and disease-modifying osteoarthritis drugs (DMOARDs). Gastrointestinal side effects are the most common, and often emergency side effects among the side effects of long-term treatment with NSAIDs and various gastrointestinal side effects from mild indigestion to ulceration, bleeding, and perforation have been reported (Best Practice & Research Clinical Gastroenterology 24 (2010) 121-132). The non-steroidal anti-inflammatory drugs, which are the most commonly used drugs for treatment of osteoarthritis, cause serious cardiovascular side effects and exhibit a mortality rate of 5% to 10% in severe cases. In particular, since most patients with osteoarthritis are elderly, treatment of osteoarthritis using NSAIDs is accompanied by these risks. Therefore, there is an urgent need for a safe therapeutic agent with excellent therapeutic effects to solve the above-described problems of the existing drugs for bone-related diseases.

**[Disclosure]**

**[Technical Problem]**

With this background, the present inventors have found that a fusion protein including a GLP-1 receptor agonist and an anti-OSCAR antibody has excellent therapeutic effects on arthritis, thereby completing the present invention.

**[Technical Solution]**

An object of the present invention is to provide a fusion protein including a glucagon-like peptide-1 (GLP-1) receptor agonist and an anti-osteoclast-associated receptor (OSCAR) antibody.

Another object of the present invention is to provide a pharmaceutical composition for preventing or treating arthritis including the fusion protein as an active ingredient.

Another object of the present invention is to provide a food composition for preventing or alleviating arthritis including the fusion protein as an active ingredient.

Another object of the present invention is to provide a health functional food composition for preventing or alleviating arthritis including the fusion protein as an active ingredient.

Another object of the present invention is to provide a method of preventing or treating arthritis, the method including administering the pharmaceutical composition to an individual.

Another object of the present invention is to provide a use of the fusion protein for preventing, alleviating, or treating arthritis.

Another object of the present invention is to provide a use of the pharmaceutical composition including the fusion protein for preventing or treating arthritis.

Another object of the present invention is to provide a use of the food composition including the fusion protein for preventing or alleviating arthritis.

Another object of the present invention is to provide a use of the health functional food composition for preventing or alleviating arthritis.

**[Advantageous Effects]**

The fusion protein including a GLP-1 receptor agonist and an anti-OSCAR antibody of the present invention may be widely used for effective treatment of arthritis due to excellent effects on protecting cartilage and relieving pain.

**[Brief Description of Drawings]**

FIG. 1 is a schematic diagram illustrating a structure of a fusion protein PF1803.
FIG. 2 shows results of SDS-PAGE and western blot performed to identify PF1803 protein.
FIG. 3 shows results of ELISA performed to identify a structure of PF1803 protein.
FIG. 4 shows inhibitory effects of PF1803 on apoptosis in apoptosis-induced chondrocytes.
FIG. 5 shows effects of PF1803 on decreasing expression levels of caspase 3 and caspase 8 in apoptosis-induced chondrocytes.
FIG. 6 shows effects of PF1803 on decreasing an expression level of MMP3 and increasing an expression level of Aggrecan in apoptosis-induced chondrocytes.
FIG. 7 shows histological analysis results to confirm effects of PF1803 on protecting cartilage in an arthritis animal model.
FIG. 8 shows weight bearing to confirm effects of PF1803 on relieving pain in an arthritis animal model.

**[Best Mode]**

Hereinafter, the present invention will be described in detail. Meanwhile, each description and embodiment disclosed in the present invention may be applied herein to describe different descriptions and embodiments. In other words, all combinations of various components disclosed in the present invention are included within the scope of the present invention. Furthermore, the scope of the present invention should not be limited by the detailed description provided below.

Also, those skilled in the art will recognize or be able to ascertain, using no more than routine experimentation, many equivalents to specific embodiments of the present invention. Such equivalents are intended to be encompassed in the scope of the following claims.

An aspect of the present invention to achieve the above-described objects provides a fusion protein including a glucagon-like peptide-1 (GLP-1) receptor agonist and an anti-osteoclast-associated receptor (OSCAR) antibody.

As used herein, the term "GLP-1 receptor agonist" refers to a protein capable of binding to a receptor of glucagon-like peptide-1 (GLP-1), which is a gastrointestinal hormone derived from a transcript of a glucagon gene and may reduce a blood sugar level. The GLP-1 receptor agonist is not particularly limited as long as it selectively stimulates the GLP-1 receptor to have a signaling pathway similar to that of GLP-1, and may include, for example, GLP-1 and derivatives thereof. The GLP-1 derivatives may be prepared by way of one of substitution, addition, deletion, and modification of some amino acids of GLP-1, or any combination thereof. These GLP-1 derivatives may be any substance well known in the art, e.g., liraglutide, exendin-4, lixisenatide, dulaglutide, and albiglutide.

As used herein, the term "osteoclast-associated receptor (OSCAR)" refers to a cell surface receptor belonging to the leukocyte receptor complex family and including two immunoglobulin (Ig) domains. The OSCAR protein or fragments thereof of the present invention may be derived from humans or mice. Genetic information such as amino acid sequences of the human- or mouse-derived OSCAR protein and nucleotide sequences encoding the same may be available via a known database such as GenBank of the National Center for Biotechnology Information (NCBI), without being limited thereto.

As used herein, the term "antibody" refers to a protein molecule capable of specifically binding to an antigenic site of a protein or peptide molecule. After obtaining a protein encoded by a marker gene by cloning each gene into an expression vector using any known method, the antibody may be prepared from the protein using any method well known in the art. The antibody consists of two light chains and two heavy chains, and each chain includes a variable domain having a variable amino acid sequence and a constant domain having a constant amino acid sequence. The antibody includes an antigen-binding site located at one end of a three-dimensional structure of the variable domains and formed of complementarity-determining regions, wherein three complementarity-determining regions are present in each of the light chain and the heavy chain. The complementarity-determining region is a region having particularly high amino acid sequence variability in the variable domains, and antibodies specific to various antigens may be discovered due to the high variability. Not only a full-length antibody but also antigen-binding fragments of the antibody molecule may also be included within the scope of the present invention.

As used herein, the "antibody fragment", referring to any part of an antibody, may be, for example, a scFv, a dsFv, Fab, Fab', F(ab')$_2$, a sdAb, a nanobody, or any combination thereof, and may include an antigen recognition site, but is not limited thereto. The Fab, which has a structure including the variable domains of the light chain and the heavy chain, the constant domain of the light chain, and a first constant domain (CH1 domain) of the heavy chain, includes one antigen binding site. The Fab' is different from the Fab in that the Fab' has a hinge region including at least one cysteine residue at the C-terminus of the CH1 domain of the heavy chain. The F(ab')$_2$ antibody is generated by a disulfide bond of the cysteine residue of the hinge region of the Fab'. The variable fragment (Fv) refers to a minimal fragment of an antibody including only the heavy chain variable domain and the light chain variable domain. The disulfide-stabilized Fv (dsFv) refers to a fragment in which the heavy chain variable domain is linked to the light chain variable domain via a disulfide bond, and the single-chain Fv (scFv) generally refers to a fragment in which the heavy chain variable domain is linked to the light chain variable domain by a covalent bond via a peptide linker. These antibody fragments may be obtained by using protease or constructed by genetic recombination technology. In addition, the sdAb and the nanobody are antibody fragments consisting of a single variable domain and may include, for example, an antibody fragment constructed from a naturally occurring heavy chain antibody consisting of a single variable domain (VH) and two constant domains (CH2 and CH3) via proteolysis or genetic recombination of the variable domain and a single-domain antibody fragment constructed by artificially modifying a variable domain of a light chain or heavy chain, without being limited thereto.

The anti-OSCAR antibody of the present invention is an antibody acting on an OSCAR protein and includes antibodies capable of inhibiting interaction between OSCAR and collagen.

In an embodiment of the present invention, the anti-OSCAR antibody that includes heavy chain variable domains and light chain variable domains may include at least one selected from the group consisting of:

1) an anti-OSCAR antibody or fragments thereof including a heavy chain variable domain including SEQ ID NO: 1 and a light chain variable domain including SEQ ID NO: 2;
2) an anti-OSCAR antibody or fragments thereof including a heavy chain variable domain including SEQ ID NO: 3 and a light chain variable domain including SEQ ID NO: 4; and
3) an anti-OSCAR antibody or fragments thereof including a heavy chain variable domain including SEQ ID NO: 5 and a light chain variable domain including SEQ ID NO: 6, without being limited thereto.

The fusion protein of the present invention may be an artificially synthesized protein in which the GLP-1 receptor agonist binds to the anti-OSCAR antibody, without being limited thereto.

In the fusion protein of the present invention, the GLP-1 receptor agonist may be linked to the anti-OSCAR antibody directly or via a linker or the fusion protein may further include another protein moiety, without being limited thereto. Any linking method commonly available in the art may be used without limitation in the fusion protein of the present invention, as long as the linkage does not change the structure or activity of the linked protein. The linker may be a peptidyl or non-peptidyl linker including 1 to 20 amino acids, without being limited thereto.

The fusion protein of the present invention may be prepared by binding a substance capable of increasing a half-life of a protein (e.g., GLP-1 receptor agonist and/or anti-OSCAR antibody) or by introducing a mutation to prevent degradation in the body, and any known method applicable to protein and enabling long-acting properties of the proteins may be included within the scope of the present invention. The substance capable of increasing the half-life may be selected from the group consisting of a polymer, fatty acid, cholesterol, albumin and fragments thereof, an albumin-binding substance, an antibody, an antibody fragment, an FcRn-binding substance, *in vivo* connective tissue, a nucleotide, fibronectin, transferrin, saccharide, heparin, and elastin. The FcRn-binding substance may be an immunoglobulin Fc region, without being limited thereto. It will be obvious that any protein having an amino acid sequence including deletion, modification, substitution, conservative substitution, or addition of some amino acids is within the scope of the present invention as long as the protein has activity identical or equivalent to that of the immunoglobulin Fc region.

The immunoglobulin Fc fragment may include 1 to 4 domains selected from the group consisting of CH1, CH2, CH3 and CH4 domains, and may include a hinge region. The immunoglobulin Fc fragment may be selected from the group consisting of IgG, IgA, IgD, IgE, IgM, and any combination or hybrid thereof, without being limited thereto.

Also, the immunoglobulin Fc region of the present invention includes not only a naturally occurring amino acid sequence but also a sequence variant (mutant) thereof. The amino acid sequence variant means that at least one amino acid residue of a naturally occurring amino acid sequence has a different sequence due to deletion, insertion, non-conservative substitution, conservative substation, or any combination thereof.

Specifically, the immunoglobulin Fc region may include CH2 and CH3 domains and may be a monomer or dimer, without being limited thereto. Alternatively, the immunoglobulin Fc region may be an immunoglobulin Fc region (SEQ ID NO: 9) in which asparagine at the 297th position of a human IgG1 Fc region (SEQ ID NO: 8) is substituted with alanine, without being limited thereto. In an embodiment, the immunoglobulin Fc region may have an amino acid sequence of SEQ ID NO: 8 or SEQ ID NO: 9, without being limited thereto.

In a specific embodiment, the fusion protein of the present invention may have an amino acid sequence of SEQ ID NO: 7 and may be used interchangeably with PF1803, but is not limited thereto.

The fusion protein of the present invention is characterized in that the GLP-1 receptor and the anti-OSCAR antibody may simultaneously act by including the GLP-1 receptor agonist and the anti-OSCAR antibody. The fusion protein may have a long-lasting therapeutic effect in the body by further inclusion of a half-life-increasing substance. Therefore, the fusion protein of the present invention may be an excellent therapeutic agent for a target disease, particularly arthritis, based on simultaneous action of the GLP-1 receptor and the anti-OSCAR antibody and the increased half-life.

Another aspect of the present invention provides a pharmaceutical composition for preventing or treating arthritis including the fusion protein as an active ingredient.

The fusion protein is as described above.

As used herein, the term "arthritis" refers to a disease causing inflammation and pain occurring due to damage to bones and ligaments constituting joints resulting from destruction or degenerative changes in cartilage that protects the joints, and is also called osteoarthritis. The arthritis of the present invention may include degenerative arthritis, osteochondritis dissecans, articular ligament injuries, meniscal injuries, joint malalignment, avascular necrosis, rheumatoid arthritis, juvenile idiopathic arthritis, and arthritis caused by trauma, inflammation, or infection, specifically degenerative arthritis or rheumatoid arthritis, without being limited thereto. The pharmaceutical composition of the present invention may have effects on delaying cartilage destruction or relieving pain to thereby have preventive or therapeutic effects on arthritis, without being limited thereto.

As used herein, the term "prevention" refers to all actions that inhibit or delay the onset of arthritis or by administering the composition of the present invention. The term "treatment" refers to all actions that ameliorate or beneficially change symptoms of arthritis by administering the composition of the present invention.

The pharmaceutical composition of the present invention may further include a pharmaceutically acceptable carrier, excipient, or diluent. The pharmaceutically acceptable carrier, excipient, or diluent may be one which does not occur naturally. Specifically, the composition may be formulated into oral dosage forms, such as powders, granules, tablets, capsules, suspensions, emulsions, syrups, and aerosols, formulations for external use, suppositories, and sterile injection solutions. In the present invention, examples of the suitable carrier, excipient, or diluent that may be included in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil. For formulations, a diluent or excipient commonly used in the art such as a filler, an extender, a binder, a humectant, a disintegrant, and a surfactant may be used. Solid formulations for oral administration may include tablets, pills, powders, granules, capsules, and the like. The solid formulations may be prepared by mixing the composition with at least one excipient, such as starch, calcium carbonate, sucrose, lactose, or gelatin. A lubricant such as magnesium stearate and talc may also be used in addition to a simple excipient. Liquid formulations for oral administration may be suspensions, formulations for internal use, emulsions, syrups, or the like, and may include various excipients such as a humectant, a sweetener, a fragrance, and a preservative in addition to a simple diluent such as water and liquid paraffin. Formulations for parental administration may include sterile aqueous solutions, non-aqueous solvents, suspensions, emulsions, lyophilizates, suppositories, and the like. The non-liquid solvents and suspensions may be propylene glycol, polyethylene glycol, vegetable oils such as olive oil, injectable esters such as ethyl oleate, or the like. Bases for the suppositories may include Witepsol, Macrogol, Tween 61, cacao butter, laurin butter, glycerogelatin, and the like.

Another aspect of the present invention provides a food composition for preventing or alleviating arthritis including the fusion protein as an active ingredient.

Another aspect of the present invention provides a health functional food composition for preventing or alleviating arthritis including the fusion protein as an active ingredient.

The fusion protein, arthritis, and prevention are as described above.

As used herein, the term "alleviation" refers to all actions that ameliorate or beneficially change symptoms of an individual having a disease and suspected of having the disease by using the composition.

As used herein, the term "food" includes all foods in the ordinary sense, such as meats, sausages, breads, chocolates, candies, snacks, cookies, pizzas, ramen, other noodles, gums, dairy products including ice cream, various kinds of soups, beverages, teas, drinks, alcoholic beverages, vitamin complexes, and health functional food, and is not particularly limited as long as the food includes the fusion protein of the present invention. The food composition may be prepared by adding any ingredients and components commonly used in the art, and types thereof are not particularly limited. For example, the food composition, like other foods, may further include various herb extracts, sitologically acceptable food auxiliary additives, or natural carbohydrates, but is not limited thereto. The amount of an effective ingredient may be appropriately determined according to the purpose of use.

As used herein, the term "health functional food", being the same as food for special health use (FoSHU), refers to a food prepared using a particular ingredient as a raw material or a food manufactured or processed by extracting, concentrating, purifying, or mixing a particular ingredient contained in food and using the ingredient as a raw material for the purpose of health supplementation. The health functional food is designed and processed to sufficiently exert body-regulatory functions, such as biodefense, biorhythm control, disease prevention, and recovery from disease, by way of the ingredient, and the health functional food composition may perform functions related to disease prevention or recovery from disease. The health functional food of the present invention may be used interchangeably with any known terms in the art such as functional food.

Another aspect of the present invention provides a method of preventing or treating arthritis, the method including administering the pharmaceutical composition to an individual.

As used herein, the term "individual" may include mammals such as mice, livestock, and humans and farmed fish which have arthritis or a risk of developing arthritis, without limitation.

The pharmaceutical composition of the present invention may be administered via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route using an administration method commonly used in the art, but is not limited thereto.

An appropriate dose of the pharmaceutical composition of the present invention may be determined by a doctor within the scope of sound medical judgment in a bolus or in multiple doses. However, for the purpose of the present invention, it is preferred that a specific therapeutically effective amount for a particular patient is differently applied depending on various factors including the type and extent of a response to be achieved, a specific composition including whether other formulations are used according to the case, age, body weight, general health status, gender, and diet of the patient, administration time, administration route, excretion rate of the composition, and duration of treatment.

Another aspect of the present invention provides a use of the fusion protein for preventing, alleviating, or treating arthritis.

Another aspect of the present invention provides a use of the pharmaceutical composition including the fusion protein for preventing or treating arthritis.

Another aspect of the present invention provides a use of the food composition including the fusion protein for preventing or alleviating arthritis.

Another aspect of the present invention provides a use of the health functional food composition for preventing or alleviating arthritis.

The fusion protein, pharmaceutical composition, food composition, health functional food composition, arthritis, prevention, alleviation, and treatment are as described above.


**[Mode for Invention]**

Hereinafter, the present invention will be described in more detail with reference to the following examples. However, the following examples are merely presented to exemplify the present invention, and the scope of the present invention is not limited thereto.


**Example 1. Preparation and Verification of Fusion Protein**

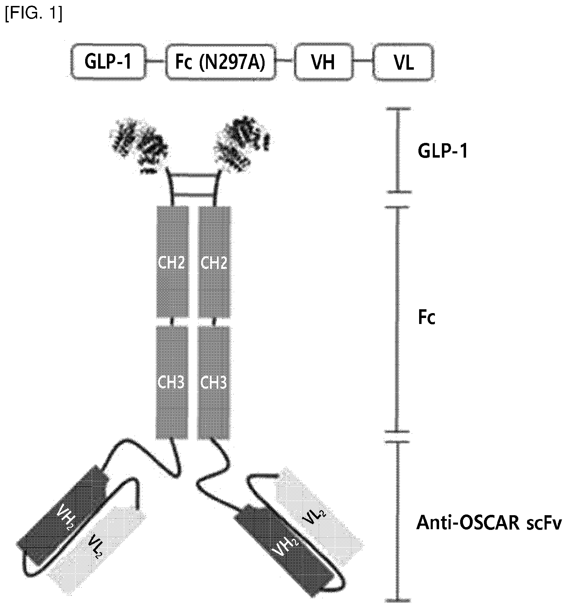

A fusion protein (PF1803; SEQ ID NO: 7) including a GLP-1 receptor agonist and an anti-OSCAR antibody was synthesized as GLP-1-Fc-VH-VL (FIG. 1). The synthesized PF1803 was cloned into a pcDNA3.1 vector using a Nhe1/HindIII site. CHO-S cells were transfected with the cloned vector, and a supernatant was collected therefrom on the 3$^{rd}$ day, and then the cells and floating substances were removed by centrifugation using a 0.22 $\mu$m filter, followed by purification using protein A beads (Cytiva, HiTrap Protein A).

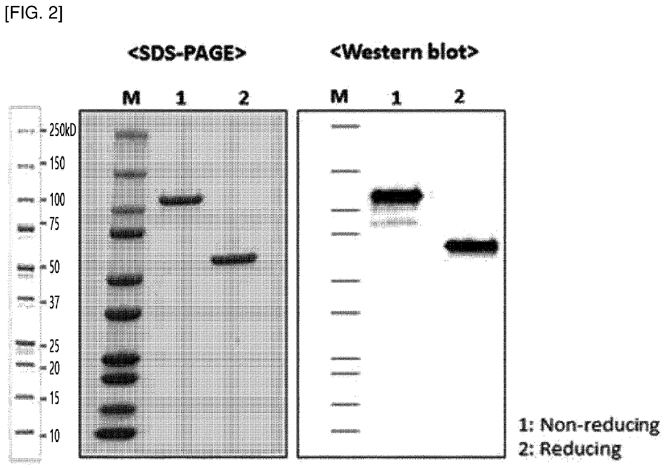

Preparation of PF1803 was confirmed by western blotting using SDS-PAGE gel and Anti-GLP1 Ab (Abcam) under non-reducing and reducing conditions (FIG. 2).

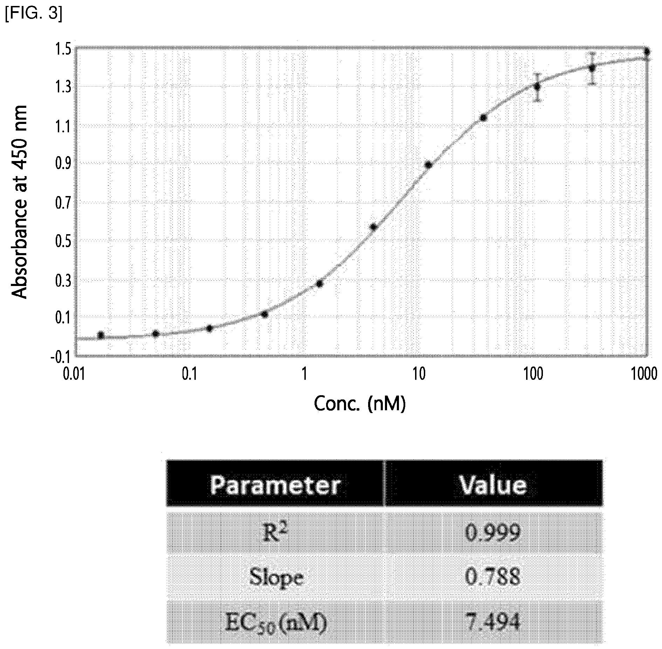

Human OSCAR protein was aliquoted into an immuno-96 microwell plate in an amount of 300 ng/well using a coating buffer and immobilized overnight at 4 °C. Phosphate buffered saline (PBST) containing 5% skim milk was added thereto in an amount of 20 $\mu$L/well, followed by reaction at 37°C for 1 hour to block non-specific binding. Thereafter, the PF1803 was diluted by a 3-fold serial dilution from the highest concentration of 1000 nM to 0.017 nM, and 100 $\mu$L of the diluted PF1803 was aliquoted into each well, followed by reaction at 37°C for 1 hour. The plate was washed three times with 200 $\mu$L of PBST, and mouse anti-GLP1 antibody (abcam) diluted to a ratio of 1:2000 was aliquoted thereinto in an amount of 100 $\mu$L/well, followed by reaction at 37°C for 1 hour. After washing the plate three times with PBST, anti-mouse IgG-HRP (Millipore) diluted to a ratio of 1:3000 was added thereto in an amount of 100 $\mu$L/well, followed by reaction at 37°C for 1 hour, and then the plate was washed three times with PBST. Subsequently, a TMB (BD) solution was added thereto in an amount of 100 $\mu$L/well, followed by reaction at room temperature for 5 minutes, and then the reaction was terminated using 100 $\mu$L of 1 N HCl. Finally, absorbance was measured at 450 nm using a spectrophotometer (Spectrmax iD3, Molecular device). As a result, an $EC_{50}$ value of 7.494 nM was confirmed (FIG. 3), and thus it was confirmed that the produced PF1803 protein binds to both OSCAR and a GLP-1 antibody.

## Example 2. MIA-induced Arthritis Model

An arthritis animal model used in the present invention is a model prepared by inducing osteoarthritis by injecting monosodium iodoacetate (MIA) into a joint cavity. MIA induces activation of matrix metalloproteinase (MMP) and inhibits synthesis of proteoglycan in cartilage to cause necrosis of chondrocytes, thereby causing symptoms similar to those of degenerative arthritis in patients.

Specifically, monosodium iodoacetate (MIA, I2512, Sigma, Poole, UK) was dissolved in saline for injection at concentrations of 20 mg/mL and 60 mg/mL for preparation of the experiment on the experiment start day (day 0). On the experiment start day, animals were classified into groups and anesthetized using isoflurane as an inhalational anesthetic agent in an anesthesia chamber, and 50 μL (MIA 1, 3 mg/body) of MIA was injected into the right articulatio genu via the infrapatellar ligament using a 26.5-gauge 1 cc syringe. Experimental groups and administered substances are shown in Table 1 below.

Table 1

| Group | Test substance | Dose | Administration method | Dosing interval |
|---|---|---|---|---|
| G1 | Vehicle | - | LA | Once a week |
| G2 | MIA + Vehicle (PBS) | - | LA | |
| G3 | MIA + PF1803 | 1.0 mg/kg | LA | |
| G4 | MIA + PF1803 | 2.0 mg/kg | LA | |
| G5 | MIA + GLP-1 analog (PF1801) + Anti-OSCAR | GLP-1 analog 0.5 mg/kg + Anti-OSCAR 0.5 mg/kg | LA | |
| G6 | MIA + Celecoxib | 50 mg/kg | Oral | Every day |

After inducing osteoarthritis by MIA, test substance-administered groups G3 to G6 were homogenized with a vehicle at a preset dose and administered into the articulatio genu once a week at a dose of 1 mL. Only the vehicle was administered to the articulatio genu of a normal control and an excipient control once a week in the same amount on the same administration schedule as for administration of the test substance. Celecoxib, which is a positive control, was orally administered at a predetermined dose once a day after being homogenized with a vehicle.

## Example 3. Inhibitory Effect on Apoptosis of Chondrocytes

Example 3-1. Inhibition of Apoptosis of Chondrocytes

To induce apoptosis of chondrocytes, chondrocytes isolated from cartilage of mice were used. The mouse chondrocytes were treated with IL-1β in an amount of 10 ng/mL and treated with OSCAR-binding triple-helical peptide (OSC, SEQ ID NO: 10), which induces a signal in a primary cell expressing OSCAR to evaluate anti-OSCAR antibody-related effects.

After treatment with IL-1β and OSC, cell viabilities of chondrocytes respectively treated with the GLP1-Fc (fusion protein in which GLP-1 and the Fc region of an antibody were fused), anti-OSCAR antibody, or PF1803 were measured.

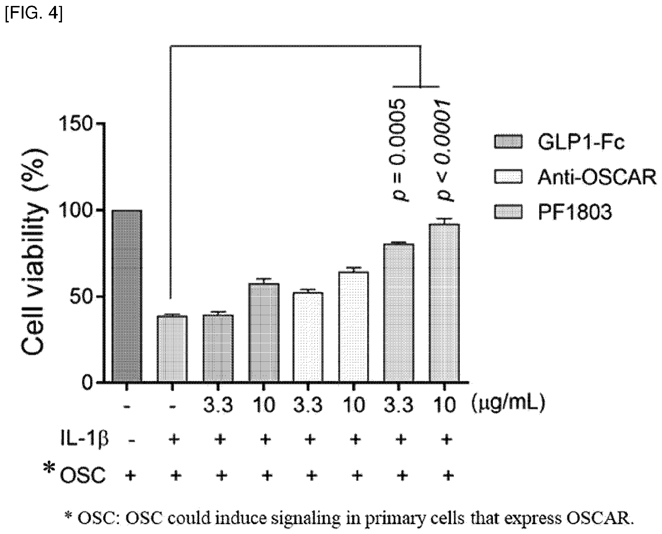

As a result, it was confirmed that apoptosis of chondrocytes treated with PF1803 was significantly inhibited in a concentration-dependent manner compared with the anti-OSCAR antibody-administered group and the GLP-1-administered group (FIG. 4).

## Example 3-2. Apoptosis Pathway (Decrease in Activity of Caspase 3 and Caspase 8)

In Example 3-1, activities of caspase 3 and caspase 8 of chondrocytes respectively treated with GLP1-Fc, anti-OSCAR antibody, or PF1803 after being treated with IL-1β and OSC were measured. Since caspase 3 and caspase 8 are proteins indicating apoptosis, the activities thereof were measured and compared.

As a method of measuring cleavage activities of caspase 3 and caspase 8, colorimetric assay kits for caspase 3 or caspase 8 were used (caspase 3 kit (Biovision K106) and caspase 8 kit (Biovision K113)).

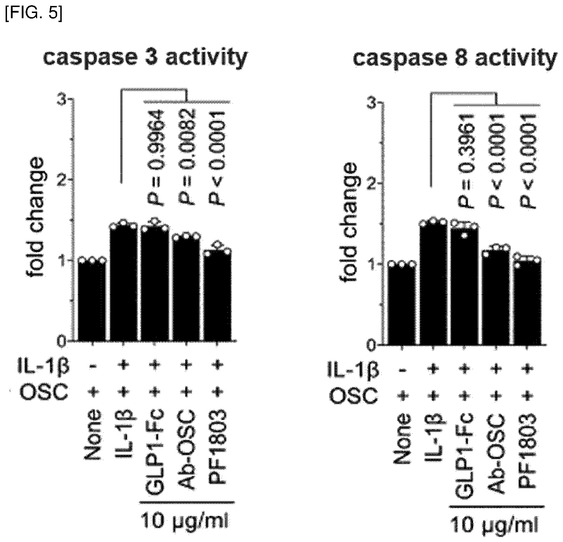

As a result, it was confirmed that the activities of caspase 3 and caspase 8 were significantly reduced in the chondrocytes treated with the PF1803 when compared with the anti-OSCAR antibody-administered group and the GLP-1-administered group (FIG. 5). This indicates that PF1803 has excellent effects on delaying cartilage destruction and improving cartilage regeneration.

**Example 3-3. Apoptosis Pathway (Decrease in Expression of MMP3 and Increase in Expression of Aggrecan)**

In Example 3-1, expression levels of MMP3 and Aggrecan of chondrocytes respectively treated with GLP1-Fc, anti-OSCAR antibody, or PF1803 after being treated with IL-1β and OSC were measured by western blotting.

Specifically, since matrix metalloproteinase-3 (MMP3) is a catabolic marker, an increase in MMP3 is an indicator of destruction of chondrocytes. Since Aggrecan is an anabolic marker, an increase in Aggrecan is an indicator of regeneration of chondrocytes. Therefore, expression levels of MMP3 and Aggrecan were measured.

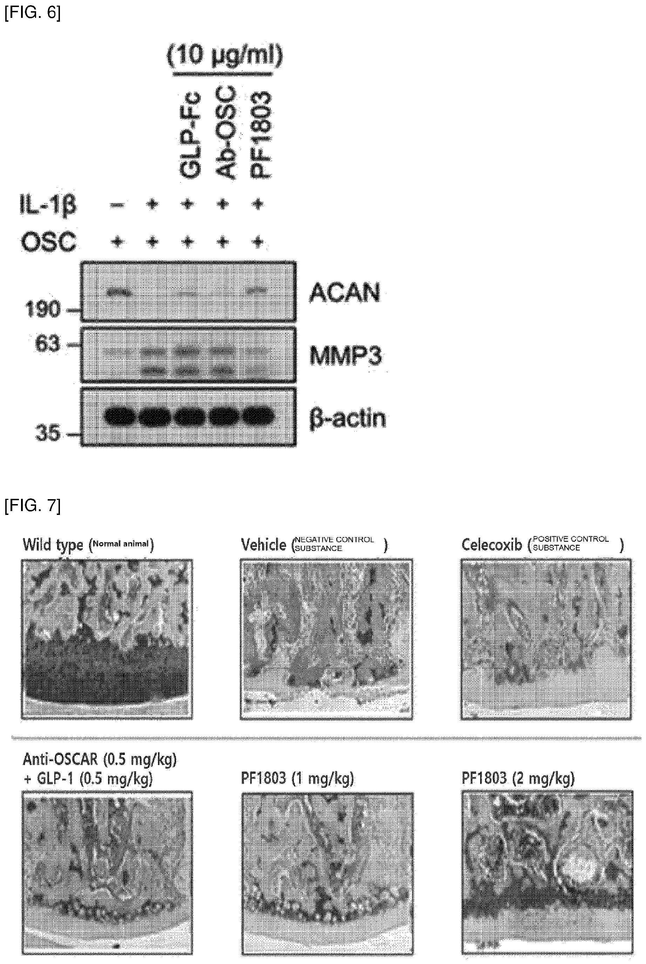

As a result, it was confirmed that the expression level of MMP3 was significantly reduced and the expression level of Aggrecan was significantly increased in the chondrocytes treated with the PF1803 when compared with the anti-OSCAR antibody-administered group and the GLP-1-administered group (FIG. 6). This indicates that PF1803 has excellent effects on delaying cartilage destruction and improving cartilage regeneration.

**Example 4. Effect of PF1803 on Delaying Cartilage Destruction**

Joints of the respective groups of the animal model were collected to prepare paraffin blocks. In order to observe the degrees of destruction of cartilage tissue and proliferation of inflammatory cells, H&E and Safranin O were used for the analysis of all cases. Difference from the control was analyzed by obtaining OARSI and Mankin scores based on the Safranin O staining results.

As a result, the cartilage of the animals administered with the positive control was completely destroyed. Upon comparison with the group co-administered with the anti-OSCAR antibody and the GLP-1, excellent cartilage destruction-delaying effects were confirmed in the PF1803-administered animals (FIG. 7).

**Example 5. Pain Relieving Effect of PF1803**

A weight bearing measurement test is a test of measuring a difference of weight bearing (or weight distribution) between a normal hind limb (left) and an arthritisinduced hind limb (right) caused by pain of the hind limb knee in which arthritis is induced on one side. Weights of both hind limbs were measured using an Incapacitance meter (Model 600, IITC, USA), which is a device for measuring weight bearing. Since the load may change according to the posture of the animal's feet, each animal was accurately positioned in the holder such that both feet were located symmetrically by one person in charge of setting the posture of the animal to reduce error as much as possible. When each animal was accurately positioned in the holder, the device was operated such that the measurement was performed twice for 5 seconds for each, and the average was used as the weight bearing (g). The measurement was performed by obtaining baseline values by measuring the weights before inducing arthritis by MIA administration (day 0) in an excipient control, an experimental group, and a positive control group, classifying the animals into groups before administering a test substance (day 3), and measuring the weights twice a week at a preset time. The results of the weight bearing test were analyzed by using the following equation by converting the results into a weight bearing ratio.

$$\text{Weight bearing ratio} = \text{weight of right hind limb} / (\text{weight of right hind limb} + \text{weight of left hind limb}) \times 100$$

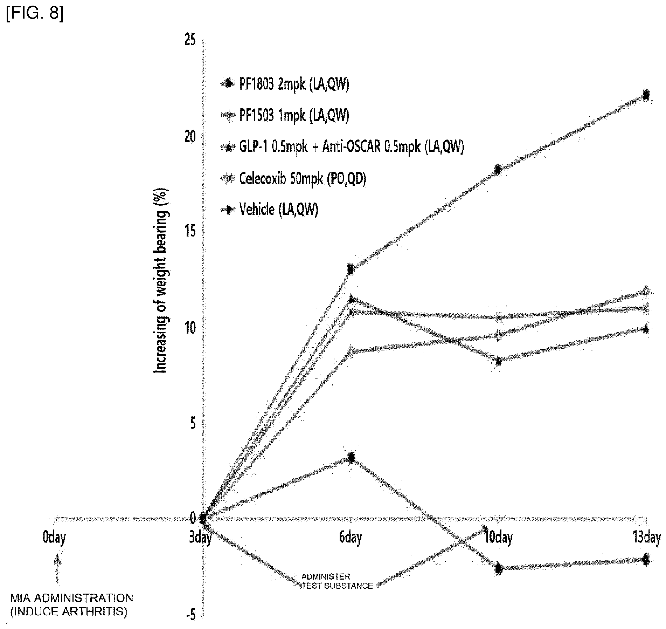

As a result, it was confirmed that the PF1803-administered group had excellent pain relieving effects compared to the positive control, or the group co-administered with the anti-OSCAR antibody and the GLP-1 (FIG. 8).

That is, these results indicate that the fusion protein including a GLP-1 receptor agonist and an anti-OSCAR antibody of the present invention may be used to prevent or treat arthritis.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

**Claims**

1. A fusion protein comprising a glucagon-like peptide-1 (GLP-1) receptor agonist and an anti-osteoclast-associated receptor (OSCAR) antibody.

2. The fusion protein of claim 1, wherein the anti-OSCAR antibody including heavy chain variable domains and light chain variable domains comprises at least one selected from the group consisting of:

   1) an anti-OSCAR antibody or fragments thereof including a heavy chain variable domain including SEQ ID NO: 1 and a light chain variable domain including SEQ ID NO: 2;
   2) an anti-OSCAR antibody or fragments thereof including a heavy chain variable domain including SEQ ID NO: 3 and a light chain variable domain including SEQ ID NO: 4; and
   3) an anti-OSCAR antibody or fragments thereof including a heavy chain variable domain including SEQ ID NO: 5 and a light chain variable domain including SEQ ID NO: 6.

3. The fusion protein of claim 1, wherein the GLP-1 receptor agonist is GLP-1 or a derivative thereof.

4. The fusion protein of claim 1, wherein the fusion protein further comprises a half-life-increasing substance.

5. The fusion protein of claim 4, wherein the half-life-increasing substance is an immunoglobulin Fc region.

6. The fusion protein of claim 5, wherein asparagine at position 297 of the immunoglobulin Fc region is substituted with alanine.

7. The fusion protein of claim 1, wherein the fusion protein comprises SEQ ID NO: 7.

8. A pharmaceutical composition for preventing or treating arthritis, comprising the fusion protein according to any one of claims 1 to 7 as an active ingredient.

9. The pharmaceutical composition of claim 8, wherein the arthritis is degenerative arthritis or rheumatoid arthritis.

10. The pharmaceutical composition of claim 8, wherein the pharmaceutical composition delays cartilage destruction or relieves pain.

11. A food composition for preventing or alleviating arthritis, comprising the fusion protein according to any one of claims 1 to 7 as an active ingredient.

12. A health functional food composition for preventing or alleviating arthritis, comprising the fusion protein according to any one of claims 1 to 7 as an active ingredient.

13. A method of preventing or treating arthritis, the method comprising administering the pharmaceutical composition of claim 8 to an individual other than a human.

[FIG. 1]

[FIG. 2]

<SDS-PAGE>  <Western blot>

1: Non-reducing
2: Reducing

[FIG. 3]

| Parameter | Value |
|---|---|
| $R^2$ | 0.999 |
| Slope | 0.788 |
| $EC_{50}$ (nM) | 7.494 |

[FIG. 4]

\* OSC: OSC could induce signaling in primary cells that express OSCAR.

[FIG. 5]

[FIG. 6]

[FIG. 7]

| Wild type (Normal animal) | Vehicle (NEGATIVE CONTROL SUBSTANCE) | Celecoxib (POSITIVE CONTROL SUBSTANCE) |

| Anti-OSCAR (0.5 mg/kg) + GLP-1 (0.5 mg/kg) | PF1803 (1 mg/kg) | PF1803 (2 mg/kg) |

[FIG. 8]

## INTERNATIONAL SEARCH REPORT

| International application No. |
| --- |
| **PCT/KR2021/009934** |

### A. CLASSIFICATION OF SUBJECT MATTER

**C07K 14/605**(2006.01)i; **C07K 16/28**(2006.01)i; **A61K 38/00**(2006.01)i; **A61P 19/02**(2006.01)i; **A23L 33/18**(2016.01)i

According to International Patent Classification (IPC) or to both national classification and IPC

### B. FIELDS SEARCHED

Minimum documentation searched (classification system followed by classification symbols)

C07K 14/605(2006.01); A61K 39/395(2006.01); C07K 16/18(2006.01); C07K 16/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GLP-1(glucagon like peptide-1), 항-오스카(anti-osteoclast-associated receptor), 항체(antibody), 융합 단백질(fusion protein), 반감기(half-life), 면역글로불린 Fc(immunoglobulin Fc), 관절염(arthritis)

### C. DOCUMENTS CONSIDERED TO BE RELEVANT

| Category* | Citation of document, with indication, where appropriate, of the relevant passages | Relevant to claim No. |
| --- | --- | --- |
| A | QUE, Qihong et al. The GLP-1 agonist, liraglutide, ameliorates inflammation through the activation of the PKA/CREB pathway in a rat model of knee osteoarthritis. Journal of Inflammtion. 2019, vol. 16, article no. 13, inner pp. 1-9. <br> See abstract; and inner pages 2-8. | 1-13 |
| A | WO 2013-011063 A1 (NOVO NORDISK A/S) 24 January 2013 (2013-01-24) <br> See abstract; and claims 1-12. | 1-13 |
| A | CHEN, Jian et al. Glucagon-like peptide-1 receptor regulates endplasmic reticulum stress-induced apoptosis and the associated inflammatory response in chondrocytes and the progression of osteoarthritis in rat. Cell Death and Disease. 2018, vol. 9, article no. 212, inner pp. 1-14. <br> See abstract; and inner pages 3-12. | 1-13 |
| A | US 2014-0056918 A1 (NOVO NORDISK A/S) 27 February 2014 (2014-02-27) <br> See abstract; and claims 1-17. | 1-13 |
| A | SOLTANI, N. et al. In vivo expression of GLP-1/IgG-Fc fusion protein enhances beta-cell mass and protects against streptozotocin-induced diabetes. Gene Therapy. 2007, vol. 14, pp. 981-988. <br> See abstract; and pages 982-985. | 1-13 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 November 2021** | **24 November 2021** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2021/009934**

**C.     DOCUMENTS CONSIDERED TO BE RELEVANT**

| Category* | Citation of document, with indication, where appropriate, of the relevant passages | Relevant to claim No. |
|---|---|---|
| A | PARK, Doo Ri et al. Osteoclast-associated receptor blockade prevents articular cartilage destruction via chondrocyte apoptosis regulation. Nature Communications. 28 August 2020 (online publication date), vol. 11, article no. 4343, inner pp. 1-11.<br>    See abstract; and inner pages 2-9.<br>    (This document is a document declaring exceptions to lack of novelty by the applicant.) | 1-13 |

Form PCT/ISA/210 (second sheet) (July 2019)

**INTERNATIONAL SEARCH REPORT**

International application No.

**PCT/KR2021/009934**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2021/009934**

| Patent document cited in search report | | | Publication date (day/month/year) | Patent family member(s) | | | Publication date (day/month/year) |
|---|---|---|---|---|---|---|---|
| WO | 2013-011063 | A1 | 24 January 2013 | WO | 2013-011061 | A1 | 24 January 2013 |
| US | 2014-0056918 | A1 | 27 February 2014 | WO | 2013-011059 | A1 | 24 January 2013 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Best Practice & Research Clinical Gastroenterology,* 2010, vol. 24, 121-132